# EUROPEAN PATENT APPLICATION

(11) **EP 4 451 273 A1**
(43) Date of publication of application: **23.10.2024**
(21) Application number: 23305584.7
(22) Date of filing: 17.04.2023
(51) Int. Cl.: G16B 15/10, G16B 15/30, G16B 20/30, G16B 35/20, G16B 40/30, G16B 40/20

(54) **MACHINE LEARNING METHOD FOR PHENOTYPE PREDICTION**

(71) Applicant: Whitelab Genomics, 91000 Evry-Courcouronnes (FR)
(72) Inventor: SERILLON, Dylan, 91000 Evry-Courcouronnes (FR)
(74) Representative: Icosa

(57) **Abstract**

The present invention relates to the generation and use of machine learning models in order to predict some characteristics of a phenotype of a biological object such as an adeno-associated virus. It also relates to the preparation of a reduced training dataset for improving the efficiency and the explicability of the machine learning models generated.

## Description

### FIELD OF INVENTION

The present invention relates to the field of complex biological processes, and more specifically to a method for preparing a reduced training dataset for training at least one machine learning model and a method for generating at least one machine learning model for predicting at least one characteristic of the phenotype of a biological object identified by at least a part of its molecular sequence.

### BACKGROUND OF INVENTION

The last decades have seen the important development of informatics lead to a revolution in the study of complex biological processes.

Applying artificial intelligence (AI) methods to existing biological datasets offers an innovative strategy to predict biological properties. Machine learning and deep learning are evolving branches of computational algorithms that use mathematical and statistical approaches to emulate human intelligence, using the surrounding environment as a learning step.

Adeno-associated virus (AAV)-based capsid libraries are growing popular as candidate selection tools for gene therapy vectors, underscored by recent advances in the study of the specific VR-VIII region. However, available data on mutations outside this region remains severely unexplored.

The present invention proposes to use machine learning models to explore and optimize the biological properties of AAV and generate suitable data on these regions, while providing high explicability.

### SUMMARY

According to a first aspect, the present disclosure relates to a computer-implemented method for preparing a reduced training dataset for training at least one machine learning model for predicting at least one characteristic of a phenotype of a biological object identified by at least one part of its molecular sequence, said method comprising:
receiving a library of molecular sequences of reference, each molecular sequence of reference being labelled with respect tosaid at least one characteristic of the phenotype of said biological object to predict;
computing, for each molecular sequence of reference, a first list of descriptors related to physicochemical and geometrical properties of at least one constructed 3D structure of said molecular sequence of reference;
computing, for each molecular sequence of reference, a second list of descriptors, said second list of descriptors comprising molecular descriptors computed directly from said molecular sequence of reference;
creating a training dataset by applying said first and second lists of descriptors to said library of molecular sequences of references;
reducing the number of descriptors by selecting from said first and second lists of descriptors at least one reduced list of descriptors according to at least one first criterion and by using said training dataset;
generating said reduced training dataset by applying said at least one reduced list of descriptors to said library of molecular sequences of reference; and
outputting said reduced training database for training at least one machine learning model for predicting at least one characteristic of a phenotype of a biological object identified by at least one part of its molecular sequence.

The library of molecular sequences of reference may be a huge dataset complicated or extremely long to analyze and understand, even for machine learning or deep learning. Thus, preparing a training dataset before training a machine learning model is advantageous for reducing the volume of the input dataset, accelerating the training of the machine learning model, focusing the training on some particular descriptors, based on a double pipeline including 3D structure and direct sequence descriptors, that may be interesting for the training depending on the final use of the machine learning model. In particular, using a training dataset obtained using said method to train a machine learning model gives accurate and more explicable predictions, thanks to carefully selected descriptors from two different pipelines (3D structure and direct molecular sequence) and their meaning.

According to other advantageous aspects of the present disclosure, said method for preparing said reduced training dataset includes one or more of the following features, taken alone or in any technically possible combination:
computing said first list of descriptors comprises, for each molecular sequence of reference:
   - constructing said least one constructed 3D structure of said molecular sequence of reference;
   - computing a first list of physicochemical and geometrical descriptors of said at least one constructed and optimized 3D structure; and
   - outputting said first list of descriptors which comprises said first lists of physicochemical and geometrical descriptors computed for each molecular sequence of reference;
computing said second list of descriptors comprises, for each molecular sequence of reference:
   - aligning said molecular sequence of reference with a full-length sequence of reference;
   - calculating molecular descriptors using said aligned molecular sequence of reference constituting said second list of descriptors; and
   - outputting said second list of descriptors which comprises all the calculated molecular descriptors;
said at least one first criterion is one of or a combination of:
   - a near-zero variance criterion, wherein near-zero variance descriptors, having a near zero variance, are excluded from said at least one reduced list of descriptors;
   - a correlation criterion, wherein, whenever at least two descriptors are correlated within a predefined threshold, only one among said at least two descriptors is included in said at least one reduced list of descriptors;
   - a high variability criterion, wherein highly variable descriptors, representative of variability, are included in said at least one reduced list of descriptors, using an unsupervised machine learning receiving said training dataset as input;
said biological object is an adeno-associated virus mutant and said library of molecular sequences is an adeno-associated virus capsid library; and/or
said at least one characteristic of the phenotype predicted is a viability of said adeno-associated virus mutant.

According to a second aspect, the present disclosure relates to a computer-implemented method for generating at least one trained machine learning model for predicting at least one characteristic of a phenotype of a biological object identified by at least one part of its molecular sequence, said method comprising:
receiving a reduced training dataset prepared according to a method for preparing said reduced training dataset according to any one of the embodiments described hereabove;
generating at least one machine learning model configured to receive said at least one part of molecular sequence identifying said biological object;
training the at least one generated machine learning model with said reduced training dataset; and
outputting said at least one trained machine learning model , and optionally outputting automatically a list of statistics at the end of the training.

As previously described, machine learning models are particularly effective in predicting biological properties and their training time can be greatly improved by preparing upstream an adapted training dataset, as well as their explicability.

According to other advantageous aspects of the present disclosure, said method for generating said at least one trained machine learning model for predicting said at least one characteristic includes one or more of the following features, taken alone or in any technically possible combination:
generating at least one machine learning model comprises generating at least two machine learning models of different types, each type of machine learning model being trained on said reduced training dataset and outputting a list of statistics representative of the performances of each trained machine learning model, the method further comprising:
   - selecting, between all the at least two trained machine learning models, at least one trained machine learning model having statistics satisfying a predetermined list of criteria computed at the end of the training step; and
   wherein the at least on trained machine learning model outputted at the end of said method is said at least one selected and trained machine learning model among the at least two trained machine learning models;
before training said at least one generated machine learning model, the method further comprises:
   - verifying that the scope of said reduced training dataset prepared is in a space of prediction of said at least one generated machine learning model;
said method further comprises optimizing said reduced training dataset, wherein an optimized list of descriptors is selected and applied to said library of molecular sequences of references to create an optimized reduced training dataset, said optimized list of descriptors being selected after a series of training sessions on untrained copies of said at least one generated machine learning model and comparisons of the list of statistics obtained after each training session; and/or
wherein said method further comprises a machine learning optimization, wherein predetermined parameters of said at least one generated machine learning model are iteratively optimized by performing a series of training sessions on untrained copies of said at least one generated machine learning model and comparisons of the list of statistics obtained after each training session.

Another aspect of the present disclosure relates to a computer-implemented method for predicting at least one characteristic of a phenotype of a biological object identified by at least one part of its molecular sequence, said method comprising:
generating at least one trained machine learning model according to a method for generating said at least one trained machine learning model according to any one of the embodiments described hereabove;
receiving and preprocessing said at least one part of molecular sequence by applying the at least one reduced list of descriptors or the optimized list of descriptors when available;
inputting the at least one preprocessed part of molecular sequence into said at least one trained machine learning model or into the at least one selected and trained machine learning model when available; and
outputting the predicted at least one characteristic of the phenotype of said biological object.

Such a method and such predictive machine learning models generated and trained are extremely valuable to the research community and to gene therapy. They save time and money by enabling to rapidly test the viability of biological objects such as adeno- associated viruses before starting to produce and test them concretely.

Another aspect of the present disclosure relates to a computer program product comprising instructions which, when the program is executed by a computer, cause the computer to automatically carry out:
a method for preparing a training dataset for training at least one machine learning model for predicting at least one characteristic of a phenotype of a biological object identified by at least one part of its molecular sequence according to any one of the embodiments described hereabove; or
a method for generating at least one machine learning model for predicting at least one characteristic of a phenotype of a biological object identified by at least one part of its molecular sequence according to any one of the embodiments described hereabove; or
a method for predicting at least one characteristic of a phenotype of a biological object identified by at least one part of its molecular sequence according to any one of the embodiments described hereabove.

Another aspect of the present disclosure relates to a non-transitory computer readable storage medium comprising instructions which, when the storage medium is read by a computer, cause the computer to carry out:
a method for preparing a training dataset for training at least one machine learning model for predicting at least one characteristic of a phenotype of a biological object identified by at least one part of its molecular sequence according to any one of the embodiments described hereabove; or
a method for generating at least one machine learning model for predicting at least one characteristic of a phenotype of a biological object identified by at least one part of its molecular sequence according to any one of the embodiments described hereabove; or
a method for predicting at least one characteristic of a phenotype of a biological object identified by at least one part of its molecular sequence according to any one of the embodiments described hereabove.

Such a non-transitory program storage device can be, without limitation, an electronic, magnetic, optical, electromagnetic, infrared, or semiconductor device, or any suitable combination of the foregoing. It is to be appreciated that the following, while providing more specific examples, is merely an illustrative and not exhaustive listing as readily appreciated by one of ordinary skill in the art: a portable computer diskette, a hard disk, a ROM, an EPROM (Erasable Programmable ROM) or a Flash memory, a portable CD-ROM (Compact-Disc ROM).

Another aspect of the present disclosure relates to a production method for producing a biological object identified by at least one part of its molecular sequence, said method comprising:
receiving said at least one part of molecular sequence identifying said biological object;
predicting a viability of said biological object according to a method for predicting at least one characteristic of a phenotype of said biological object identified by said at least one part of molecular sequence according to any one of the embodiments described hereabove, wherein said at least one characteristics of the phenotype comprises the viability; and
producing said biological object if the viability meets at least one predetermined criterion.

Advantageously, said method may further comprise testing said biological object produced to validate a list of technical effects related to said biological object, such as, for example, at least one therapeutic effect.

Another aspect of the present disclosure relates to a gene therapy treatment method with a viral vector defined by at least one part of its molecular sequence, the method comprising:
producing said viral vector according to a production method for producing a biological object identified by at least one part of its molecular sequence according to any one of the embodiments described hereabove, wherein said biological object is said viral vector;
applying said viral virus to a patient requiring said gene therapy treatment.

Since the recent advances in the study of the specific VR-VIII region adeno-associated virus (AAV)-based capsid libraries are growing popular as candidate selection tools for gene therapy vectors. The present method offers a rapid and effective means to evaluate the viability of newly designed AAV mutant for specific gene therapy treatments so it could be tested and be used as quickly as possible given the lengthy validation and marketing authorization processes existing in the medical fields.

### DEFINITIONS

In the present disclosure, the following terms have the following meanings:

"**Biological object**" refers to:
- multicellular organisms or part thereof such as, for example, plants or animals;
- cells or part thereof, such as, for example, cells of animal, plant, fungal, protist, bacterial or archaebacterial origin;
- cell-free microorganisms or parts thereof such as, for example, viruses, viral capsids; and/or
- molecules or combinations thereof such as, for example, proteins (including peptides and polypeptides),

that can be used for scientific, medical, or commercial purposes. Biological objects can be naturally occurring or created through genetic engineering or other biotechnological techniques.

"**Adeno-associated virus**" (AAV) refers to a small, non-pathogenic virus that belongs to the Parvoviridae family. AAV can infect a broad range of host cells, including both dividing and non-dividing cells, and can integrate into the host genome at a specific site on chromosome 19. AAV is characterized by its small size (about 25 nm in diameter), single-stranded DNA genome, and lack of pathogenicity or significant immune response in humans. AAV has been widely used as a vector for gene therapy, gene editing, and other biomedical applications, due to its ability to efficiently deliver transgenes to target cells, and its relative safety and low immunogenicity. AAV vectors can be engineered to carry therapeutic genes, Short hairpin RNAs (shRNAs), micro RNAs (miRNAs), or other nucleic acid sequences, and can be delivered to target tissues via various routes, such as intravenous injection, intra-tissue injection, or inhalation.

"**AAV mutants**" refer to genetic variants of natural AAV or AAV of reference (i.e. genetic variants of the AAV capsid) that have been engineered to exhibit specific properties, such as enhanced transduction efficiency, altered tropism, increased stability, or reduced immunogenicity. These mutants can be created by introducing targeted mutations into the AAV genome (more specifically mutations in the genes coding for capsid protein) using molecular biology techniques, such as site-directed mutagenesis, chimeric methodology, bio-conjugation, insertion mutagenesis, or CRISPR/Cas-mediated genome editing. The resulting AAV mutants can be used as vectors for gene therapy, gene editing, or other biomedical applications, either alone or in combination with other therapeutic agents.

**"Molecular sequence"** refers to the linear arrangement of amino acids that make up a protein. As used herein, "molecular sequence" may thus refer either to a nucleic acid sequence or, preferably, to an amino acid sequence. These molecular sequences can be used to identify and characterize biological objects, and can provide important information about their structure, function, and evolution. In the present context, a molecular sequence may particularly refer to a sequence of nucleotides or, preferably, amino acids that has been identified, isolated, and potentially modified for a specific use or application, such as a therapeutic agent like mutations in an adeno-associated virus for example.

**"Library of molecular sequences of reference",** as used herein, refers to a known set of molecular sequences (or of parts of molecular sequences), that naturally exist or have been created, and for which a phenotype is known and well identified for each molecular sequence (respectively for each part of molecular sequence).

**"Phenotype",** as used herein refers to at least one observable/assessable characteristic(s) or trait(s) of a biological object as described herein that is determined by the genetic makeup of said biological object. As used herein, genetic makeup may refer to the genome of a organism or microorganism or to a nucleic acid sequence. These characteristics or traits can include physical or functional properties, such as the viability, biodistribution, ADMET (administration distribution metabolization elimination toxicity) properties, immunogenicity, tropisms for biological target, or any other relevant biological properties.

"**Viability**" refers to the ability of a biological object practicable and which successfully performs its intended functions such as, for a virus or viral capsid, infecting target cells, delivering the therapeutic gene, and ensuring the expression of the therapeutic gene in the target cells without causing adverse effects. Predicting the viability of a biological object with a machine learning model involves training the model on a set of known molecular sequences and their corresponding viability outcomes. These outcomes can be based on various factors, such as transduction efficiency, tissue specificity, immunogenicity, and manufacturing yield. The trained machine learning model can then be used to predict the viability of new, previously untested biological objects, aiding researchers in the development of more effective and safer gene therapy biological objects. Viability is an important factor to consider when designing and optimizing biological objects for gene therapy applications for example. In the context of predicting the viability of an adeno-associated virus (AAV) capsid sequence with a machine learning model, the term "viability" may particularly refer to the ability of an AVV capable of (i) being produced after transfection of producer cells or packaging cells (such as, for example, HEK cells) with sufficient yields, to allow positive quantification by qPCR, (ii) being infectious by expression tests of a reporter gene (GFP in the case of our infectivity test) in cell lines used in GFU (green-fluorescence units) assay, and entry tests in target cells of interest thanks to variant detection by high throughput sequencing.

"**Descriptor**" refers to a quantitative representation of the physicochemical, geometrical or biological properties of a biological object identified by at least one part of molecular sequence. Descriptors can be used to characterize the structure, reactivity, and biological activity of a a biological object identified by at least one part of molecular sequence. These descriptors can include parameters such as molecular weight, solubility, electronegativity, and hydrogen-bonding capacity, among others. In the present context, a descriptor may particularly refer to a parameter or set of parameters that have been identified as being relevant to the activity or function of a biological object identified by at least one part of molecular sequence, and which may be used to design or optimize new biological object for a specific application or target.

**"Physicochemical properties"** of a molecular sequence (particularly an amino acid sequence) refer to its chemical and physical characteristics, including its structure, composition, polarity, and interactions with other molecules. These properties can play a critical role in determining the function and behavior of the biological object identified by at least one part of its molecular sequence, such as its solubility, stability, and reactivity. In the present context, the physicochemical properties of a biological object may particularly be important for understanding its potential applications and limitations, as well as for designing or modifying the biological object to improve its properties for a particular use. This information may be obtained through various experimental techniques, such as spectroscopy, chromatography, or computational modeling.

**"Geometrical properties"** of a biological object identified by at least one part of its molecular sequence refer to its three-dimensional structure and the arrangement of its constituent atoms and bonds. These properties can have a significant impact on the biological object's activity and interactions with other molecules. For example, the orientation and spatial arrangement of functional groups within a biological object can influence its binding affinity to a particular receptor or enzyme. In the present context, the geometric properties of a molecular sequence may particularly be important for understanding its structure-activity relationship and for designing or modifying the biological object to improve its properties for a particular use. These properties can be determined through experimental techniques, such as X-ray crystallography or nuclear magnetic resonance (NMR) spectroscopy, or through computational methods, such as molecular modeling or docking simulations.

**"Processor"** should not be construed to be restricted to hardware capable of executing software, and refers in a general way to a processing device, which can for example include a computer, a microprocessor, an integrated circuit, or a programmable logic device (PLD). The processor may also encompass one or more Graphics Processing Units (GPU), whether exploited for computer graphics and image processing or other functions. Additionally, the instructions and/or data enabling to perform associated and/or resulting functionalities may be stored on any processor-readable medium such as, e.g., an integrated circuit, a hard disk, a CD (Compact Disc), an optical disc such as a DVD (Digital Versatile Disc), a RAM (Random-Access Memory) or a ROM (Read-Only Memory). Instructions may be notably stored in hardware, software, firmware or in any combination thereof.

### BRIEF DESCRIPTION OF THE DRAWINGS

The present disclosure will be better understood, and other specific features and advantages will emerge upon reading the following description of particular and non-restrictive illustrative embodiments, the description making reference to the annexed drawings wherein:
**Figure 1** is a schematic representation of an embodiment of a method for preparing a reduced training dataset for training at least one machine learning model.
**Figure 2** is a schematic representation of an embodiment of a method for generating at least one trained machine learning model for predicting at least one characteristic of a phenotype of a biological object identified by at least one part of its molecular sequence.
**Figure 3** illustrates an embodiment for an optimization of a reduced training dataset that can be used in the method of **Figure 2****.**
**Figure 4** is a schematic representation of an embodiment of a method for predicting at least one characteristic of the phenotype of a biological object identified by at least one part of its molecular sequence.
**Figure 5** illustrates a device that can be used to implement one of, or all the methods illustrated in **Figure 1**, **2** and **4****.**

On the figures, the drawings are not to scale, and identical or similar elements are designated by the same references.

### DETAILED DESCRIPTION

The present description illustrates the principles of the present disclosure. It will thus be appreciated that those skilled in the art will be able to devise various arrangements that, although not explicitly described or shown herein, embody the principles of the disclosure and are included within its spirit and scope.

All examples and conditional language recited herein are intended for educational purposes to aid the reader in understanding the principles of the disclosure and the concepts contributed by the inventor to furthering the art, and are to be construed as being without limitation to such specifically recited examples and conditions.

Moreover, all statements herein reciting principles, aspects, and embodiments of the disclosure, as well as specific examples thereof, are intended to encompass both structural and functional equivalents thereof. Additionally, it is intended that such equivalents include both currently known equivalents as well as equivalents developed in the future, i.e., any elements developed that perform the same function, regardless of structure.

Thus, for example, it will be appreciated by those skilled in the art that the block diagrams presented herein may represent conceptual views of illustrative circuitry embodying the principles of the disclosure. Similarly, it will be appreciated that any flow charts, flow diagrams, and the like represent various processes which may be substantially represented in computer readable media and so executed by a computer or processor, whether or not such computer or processor is explicitly shown.

The functions of the various elements shown in the figures may be provided through the use of dedicated hardware as well as hardware capable of executing software in association with appropriate software. When provided by a processor, the functions may be provided by a single dedicated processor, a single shared processor, or a plurality of individual processors, some of which may be shared.

It should be understood that the elements shown in the figures may be implemented in various forms of hardware, software or combinations thereof. Preferably, these elements are implemented in a combination of hardware and software on one or more appropriately programmed general-purpose devices, which may include a processor, memory and input/output interfaces.

### Method for Preparing a Reduced Training Dataset

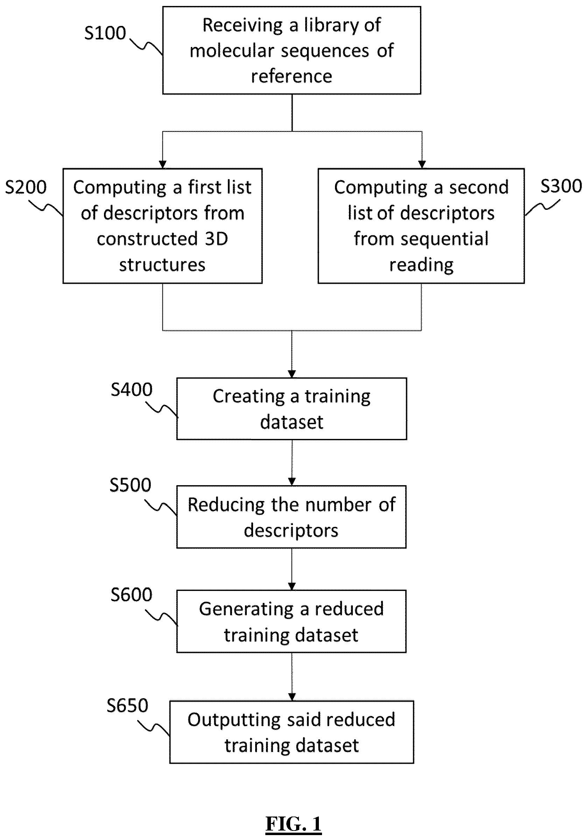

**Figure 1** illustrates an embodiment of a computer-implemented method for preparing a reduced training dataset for training at least one machine learning model for predicting at least one characteristic of a phenotype of a biological object identified by at least one part of its molecular sequence.

In some embodiment, the biological object is a virus or a part thereof like an adeno-associated virus mutant, and the library of molecular sequences is an adeno-associated virus capsid library.

In the case of genetic research and treatments, one of the characteristics of the phenotype that is interesting to predict is the viability of an adeno-associated virus capsid that can reflect its capabilities of (i) being produced after transfection of producer cells or packaging cells (such as, for example, HEK cells) with sufficient yields, to allow positive quantification by qPCR, (ii) being infectious by expression tests of a reporter gene (GFP in the case of our infectivity test) in cell lines used in GFU (green-fluorescent units) assay, and entry tests in target cells of interest thanks to variant detection by high throughput sequencing.

Generally, the viability is represented by a number, like a percentage or a real number. For example: "0" could mean that the biological object is not viable (e.g. not manufacturable and/or not operational by not being infectious for a virus), "1" could mean that the biological object is viable (e.g. meeting criteria (i) and (ii) above-cited), and "0,xx" could mean that the biological object is viable at xx%, wherein xx is an integer ranging from 1 to 99. According to this percentage, it will be interesting or not to try to produce the biological object such an AAV designed for fulfil a special function.

The method comprises seven main steps which are described here-after:
- a receiving step S100;
- a first computing step S200;
- a second computing step S300;
- a creating step S400;
- a reducing step S500;
- a generating step S600; and
- an outputting step S650.

### Receiving step S100

The receiving step S 100 consists in receiving a library of molecular sequences of reference S 100, each molecular sequence of reference being labelled with respect to said at least one characteristic of the phenotype of said biological object to predict.

Preferably, the molecular sequence comprises at least about 1 amino acids and at most 735 amino acids plus the size of any insertion made on the sequence of a natural AAV or of an AAV of reference, more preferable at least about 28 amino acids and at most 735 amino acids plus the size of any insertion made on the sequence of a natural AAV or of an AAV of reference. This molecular sequence may represent an entire capsid protein or just the sequence of the area of the capsid or protein modified and whose characteristics are to be predicted.

For example, a library of molecular sequences of reference can comprise a modification of amino acid 360-589 of natural capsids constituting a library composed by thousands capsids, mutated in several different positions.

### First computing step S200

The first computing step S200 consists in computing, for each molecular sequence of reference, a first list of descriptors related to physicochemical and geometrical properties of a constructed 3D structure of said molecular sequence of reference.

Preferably, in some embodiments, computing said first list of descriptors comprises, for each molecular sequence of reference, the following sub-steps:
constructing said at least one constructed 3D structure of said molecular sequence of reference;
computing a first list of physicochemical and geometrical descriptors of said at least one constructed 3D structure; and
outputting said first list of descriptors which comprises said first lists of physicochemical and geometrical descriptors computed for each molecular sequence.

Multiple 3D structures of the molecular sequence of reference can be reconstructed, by using AlphaFold algorithms for example. Advantageously, an optimization of the at least one constructed 3D structure may be processed by minimizing the Gibb energy, with Steepest Decent and Conjugated algorithm for example. In this case, at least one optimized 3D structure is selected and used for computing the first list of physicochemical and geometrical descriptors, said at least one optimized 3D structure corresponding to the structure having the lowest or a Gibbs free energy lower than a predetermined threshold, which generally corresponds to most stable one.

The computation of physicochemical and geometrical descriptors of the at least one constructed and optimized 3D structure, may particularly be focused on the polarizable and hydrophobic surface of the modified amino acids of the molecular sequence of reference.

For example, the physicochemical and geometrical descriptors may comprise: distance descriptors, angle descriptors, deviation descriptors, radius of gyration descriptors, any descriptor relative to the polarity of the amino acids or of a considered region of the sequence, and/or any descriptor relative to the hydrophobicity of the amino acids or of a considered region of the sequence.

### Second computing step S300

The second computing step S300 consists in computing, for each molecular sequence of reference, a second list of descriptors, said second list of descriptors comprising molecular descriptors computed directly from said molecular sequence of reference.

Preferably, in some embodiments, computing said second list of descriptors comprises, for each molecular sequence of reference, the following sub-steps:
aligning said molecular sequence of reference with a full-length sequence of reference;
calculating molecular descriptors using said aligned molecular sequence of reference constituting said second list of descriptors.

Multiple Sequence Alignment (MSA) is used to align the molecular sequence of reference, that identifies the corresponding biological object of reference, by using the closest existing biological object whose full-length molecular sequence is known. This sub-step is important to identify the invariant parts of the molecular sequence of the biological object of reference particularly when its molecular sequence comprises insertions or mutations.

Advantageously, the "Peptides" package in R may be used to theoretically calculate the molecular descriptors, by directly reading the molecular sequence of reference. For example, this package enables to compute 66 descriptors for each amino acid of a protein sequence, as well as other descriptors, such as: crucianiProperties, kideraFactors, zScales, FASGAI, tScales, VHSE, protFP, stScales, BLOSUM, MSWHIM. Other packages or platforms may also be used, like CORINA software, or DRAGON, RDKIT, MORDRED, AlvaDesc, CDK, ChemAxon, MERA, MERSY, MOPAC, for example.

It is to be noted that the first and second computing steps S200 and S300 are completely independents and can be carried out in parallel so as to gain computer processing time.

### Creating step S400

When the first and second lists of descriptors are computed, a training dataset can be created by applying said first and second lists of descriptors to the library of molecular sequences of references, so as to obtain a first reduced matrix of data to train the at least one machine learning model.

Table 1 illustrates an example of descriptors that can compose the first and second lists of descriptors.

**Table 1**

| **Descriptors** | **Description** | **Abbreviation** |
|---|---|---|
| Molecular weight | Molecular weight in [g/mol] derived from the gross formula | Weight |
| Number of hydrogen bonding acceptors | Number of hydrogen bonding acceptors derived from the sum of nitrogen and oxygen atoms in the molecular sequence | HAcc |
| Number of oxygen atom-based hydrogen bonding acceptors | Number of hydrogen bonding acceptors derived from the sum of oxygen atoms only in the molecular sequence | HAcc_O |
| Number of nitrogen atom-based hydrogen bonding acceptors | Number of hydrogen bonding acceptors derived from the sum of nitrogen atoms only in the molecular sequence | HAcc_N |
| Number of hydrogen bonding donors | Number of hydrogen bonding donors derived from the sum of N-H and O-H groups in the molecular sequence | HDon_O |
| Number of oxygen atom-based hydrogen bonding donors | Number of hydrogen bonding donors derived from the sum of O-H groups only in the molecular sequence | HDon |
| Number of nitrogen atom-based hydrogen bonding donors | Number of hydrogen bonding donors derived from the sum of N-H groups only in the molecular sequence | Hdon_N |
| Octanol/water partition coefficient (logP) | Octanol/water partition coefficient in [log units] of the molecular sequence following the XlogP | XlogP |
| Topological polar surface area | Topological polar surface area in [Å2] of the molecular sequence derived from polar 2D fragments | TPSA |
| Number of rotatable bonds | Number of open-chain, single rotatable bonds | NRotBond |
| Number of Tule of 5 violations | Number of violations of the Lipinski's rule of 5 (Weight > 500, XlogP > 5, HDon > 5, HAcc > 10) | NViolationsRo5 |
| Number of extended Rule 5 of violations | Number of violations of the extended Lipinski's rule of 5 (additional rule: number of rotatable bonds > 10) | NViolationExtsRo5 |
| Numbers of atoms | Number of all atoms in the molecular sequence (including hydrogen atoms) | NAtoms |
| Number of tetrahedral stereo centres | Number of tetrahedral chiral centres in the molecular sequence | NStereo |
| Molecular complexity | Molecular complexity according to the approach by J. Hendrickson | Complexity |
| Ring complexity | Ring complexity according to the approach by J. Gasteiger and C. Jochum | RComplexity |

### Reducing step S500

To further reduce the input used to train the at least one machine learning model and only select the most relevant descriptors, a reduced selection is made from said first and second list of descriptors according to at least one first criterion and by using the training dataset, in order to generate at least one reduced list of descriptors.

Preferably, in some embodiments, said at least one first criterion is one of or a combination of:
- a near-zero variance criterion, wherein near-zero variant descriptors, having a near zero variance, are excluded from said at least one reduced list of descriptors;
- a correlation criterion, wherein whenever at least two descriptors are correlated within a predefined threshold, only one among said at least two descriptors is included in said at least one reduced list of descriptors;
- a high variability criterion, wherein highly variable descriptors, representative of variability, are included in said at least one reduced list of descriptors, using an unsupervised machine learning receiving the training dataset as input.

Caret package may be use to delete the descriptors that have a most near-zero variance, and also to delete the most correlated descriptors.

The aim of the method is to reduce the training dataset, thus it is important to select the descriptors that best represent and cover a majority of the variety of input data. In this step, each descriptor may be used alone and/or combined with other relevant descriptors like orthogonality parameters for example.

### Generating step S600

Once the reduced list of descriptors is obtained, said reduced list of descriptors can be applied the library of molecular sequences of reference so as to generate a reduced training dataset.

### Outputting step S650

The outputting step S650 consists in outputting said reduced training database for training at least one machine learning model for predicting at least one characteristic of a phenotype of a biological object identified by at least one part of its molecular sequence.

This reduced training dataset will enable to increase the performance of the at least one machine learning models trained, while keeping a good prediction rate.

### Method for generating a machine learning model

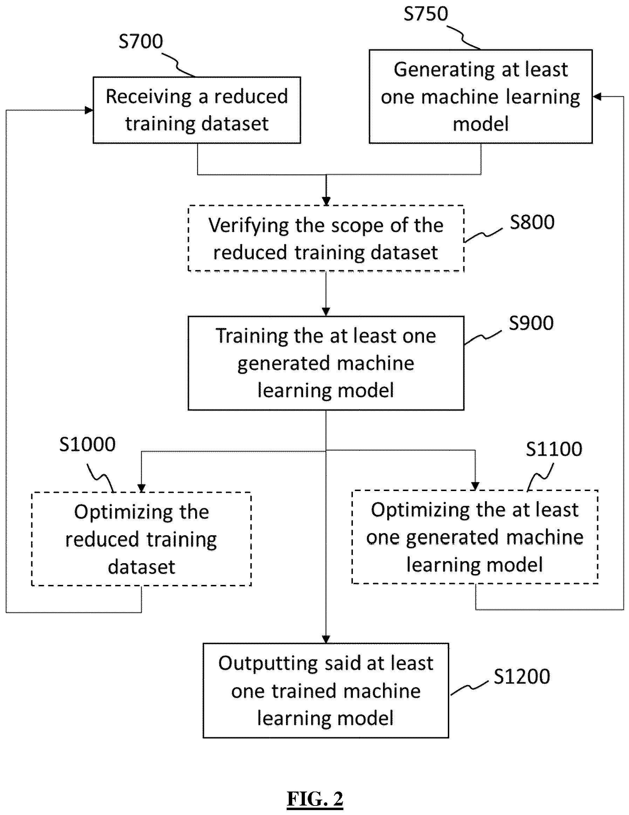

**Figure 2** illustrates an embodiment of a computer-implemented method for generating at least one machine learning model for predicting at least one characteristic of a phenotype of a biological object identified by at least one part of its molecular sequence.

This method comprises four main steps which are described here-after:
- a first receiving step S700;
- a generating step S750;
- a training step S900; and
- an outputting step S1200.

The first receiving step S700 consists in receiving a reduced training dataset prepared according to any one of the embodiments of the method for preparing said training dataset as described above.

The generating step S750 consists in generating at least one machine learning model configured to receive said at least one part of molecular sequence identifying said biological object.

Advantageously, in some embodiments, at least two machine learning models of different type are generated, and configured to receive said at least one part of molecular sequence identifying said biological object.

To ensure the convergence of the at least one generated machine learning model it is advantageous, in some embodiments, to verify, before the training step S900, that the scope of the reduced training dataset belongs to the space of the prediction of the at least one generated machine learning model S800, by using unsupervised machine learning models. To do so, the distribution of the descriptors used, represented by the percentage of variability explained by an axis, is verified.

The training step S900 consists in training each generated machine learning model with the reduced training dataset. In some embodiments, different types of machine learning model can be generated and trained on said reduced training dataset, such as but not limited to: K-Nearest Neighbours (KNN), Support Vector Machines (SVM), Random Forest (RF) or Neural Network (NNET) for example.

The outputting step S1200 consists in outputting the at least one machine learning model trained, and optionally a list of statistics (or a "confusion matrix") automatically generated at the end of the training. This list of statistics may be configured so as to improve the explicability of the result obtained when the machine learning model is used on other molecular sequences (or parts of molecular sequence) identifying unknown biological objects.

When multiple and/or different type of machine learning models are trained, at least one trained machine learning model is selected among the different machine learning models trained, and outputted at the end of said method S1200. The selection is made according to lists of statistics representatives of the performance of each trained machine learning model and a predetermined list of criteria computed at the end of the training step. These criteria may comprise: accuracy, sensitivity, specificity or prevalence for example. The at least one trained machine learning model satisfying the predetermined list of criteria is the one(s) selected and outputted.

In some embodiments, said method for generating said at least one trained machine learning model may advantageously comprise one or both of optimizing sub-steps:
- an optimization of the reduced training dataset S1000; and/or
- an optimization of the at least one generated machine learning model S1100.

### Optimization of the reduced training dataset S1000

Based on the lists of statistics automatically returned by every machine learning model at the end of the training, it is possible to identify which descriptors are the most used, which are little used and which poorly perform by giving wrong results. According to these statistics, a new list of descriptors may be selected and applied to further optimized the reduced training dataset.

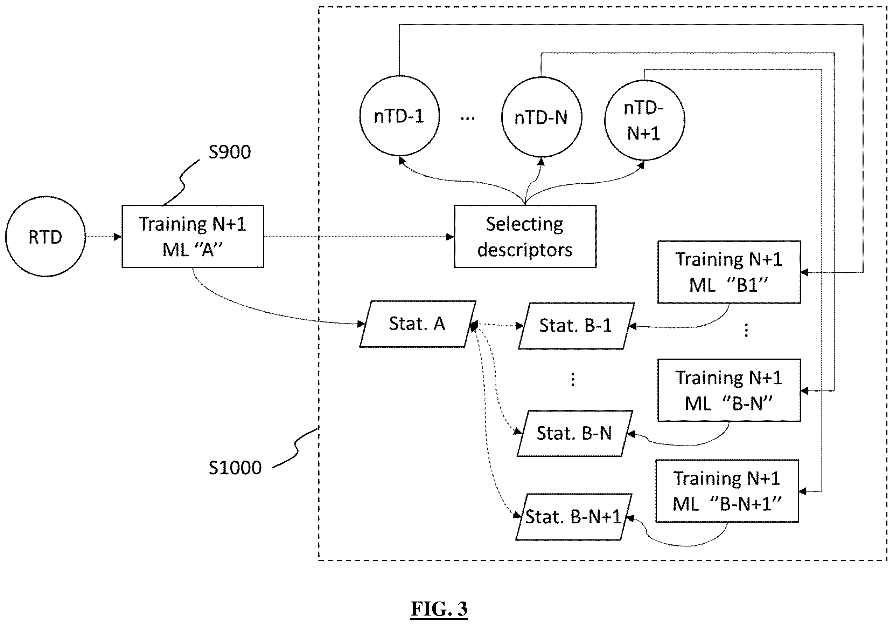

**Figure 3** illustrates an embodiment for the optimization of the reduced training dataset.

In this embodiment, a first set of N+1 machine learning models "A" are trained with the reduced training dataset RTD prepared by one of the embodiments of the method described above and following the training step S900, N being an integer greater than or equal to 0.

According to the statistics generated automatically by each trained machine learning model, a selection of descriptors is realized and applied to the library of molecular sequence of reference so as to create new training datasets nTD-1 to nTD-N+1, wherein new training datasets nTD-j corresponds to the selection of descriptors based on the statistics obtained on the trained machine learning model j, wherein j is an integer and j ∈ [1; N+1].

Then, these new training datasets nTD-1 to nTD-N+1 are individually tested by training a second set of N+1 machine learning models "B" corresponding to untrained copies of the N+1 machine learning models "A". Each statistics B-1 to B-N+1 is compared to the statistics A and the training dataset, among the new training datasets nTD1 to nTD-N+1 and the reduced training dataset, which has the best statistics is selected to be the optimized reduced training dataset. The corresponding optimized list of descriptors is also selected and outputted because it will serve to prepare the at least one part of molecular sequence identifying an unknown biological object whose phenotype is to be predicted by the machine learning model outputted at the end of step S1200.

Multiple iterations of this optimization can be performed. Furthermore, the near-zero variance, the correlated and the high variability descriptors criteria may also be used alone or in combination, and also adjusted during this optimization sub-step.

### Optimization of the at least one machine learning model S1100

In the same manner as for the optimization of the reduced training dataset S 1000, predetermined parameters of each machine learning model trained may be optimized by comparing the statistics obtained at the end of each training.

In this case, it is not the training datasets that varies between the first set of N+1 machine learning model "A" and the second set of N+1 machine learning model "B" but the settings of said predetermined parameters.

### Method for predicting a characteristic of a phenotype of a biological object

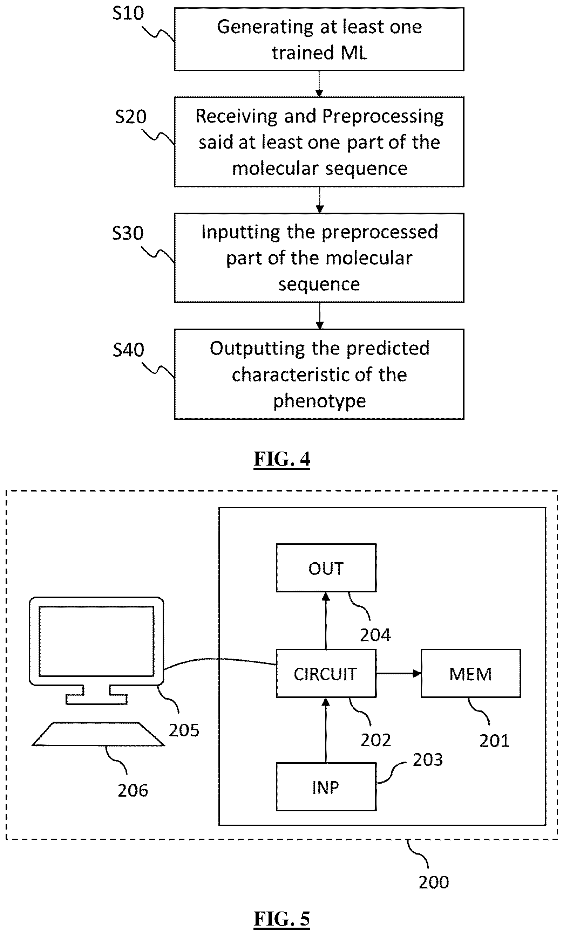

**Figure 4** illustrates an embodiment of a method for predicting at least one characteristic of a phenotype of a biological object identified by at least one part of its molecular sequence.

This method comprises the following steps:
generating at least one trained machine learning model S10 according to the method for generating said at least one trained machine learning model according to any one of the embodiments described hereabove;
receiving and preprocessing said at least one part of molecular sequence by applying the at least one reduced list of descriptors or the optimized list of descriptors when available S20;
inputting the at least one preprocessed part of molecular sequence into said at least one trained machine learning model or the at least one selected and trained machine learning model when available S30; and
outputting the predicted at least one characteristic of the phenotype of said biological object S40.

Since each predicting machine learning model generated was trained with a reduced or even optimized training dataset by selecting the best performing descriptors, it is important to preprocess the at least one part of molecular sequence that identifies the biological object which we want to predict one or more characteristics of its phenotype.

In some embodiments, this preprocessing step may advantageously be integrated into the machine learning models generated.

As described before, since the recent advances in the study of the specific VR-VIII region adeno-associated virus (AAV)-based capsid libraries are growing popular as candidate selection tools for gene therapy vectors, and the present method may offer a rapid and effective means to evaluate the viability of newly designed AAV mutant.

### Computer program product and non-transitory computer readable storage medium

The method for preparing a reduced training dataset as previously described, as well as the method for generating at least one trained machine learning model and/or the method for predicting at least one characteristic of a phenotype of a biological object identified by at least one part of its molecular sequence, may be implemented with a device 200 such as illustrated on **Figure 5****.**

In these embodiments, the device 200 comprises a computer, this computer comprising a memory 201 to store program instructions loadable into a circuit and adapted to cause a circuit 202 to carry out steps of the methods previously described when the program instructions are run by the circuit 202. The memory 201 may also store data and useful information for carrying steps of the present invention as described above.

The circuit 202 may be for instance:
- a processor or a processing unit adapted to interpret instructions in a computer language, the processor or the processing unit may comprise, may be associated with or be attached to a memory comprising the instructions, or
- the association of a processor / processing unit and a memory, the processor or the processing unit adapted to interpret instructions in a computer language, the memory comprising said instructions, or
- an electronic card wherein the steps of the invention are described within silicon, or
- a programmable electronic chip such as a FPGA chip (for « Field-Programmable Gate Array »).

The computer may also comprise an input interface 203 for the reception of input data and an output interface 204 to provide output data. Examples of input data may be the library of molecular sequences of reference or, the at least one part of a molecular sequence identifying a biological object, and output data may be a reduced training dataset, or at least one machine learning model generated and/or trained, a list of statistics, or at least one characteristic of a phenotype of a biological object, during the process of the methods.

To ease the interaction with the computer, a screen 205 and a keyboard 206 may be provided and connected to the computer circuit 202.

The device 200 may comprise a computer program product for providing the optimal sequence of actions causing the evolution of a complex system from an initial state to a final state, the computer program product comprising instructions which, when the program is executed by a computer, cause the computer to automatically carry out the steps of the methods according to any one of the above-described embodiments.

The computer program product to perform the methods as described above may be written as computer programs, code segments, instructions or any combination thereof, for individually or collectively instructing or configuring the processor or computer to operate as a machine or special-purpose computer to perform the operations performed by hardware components. In one example, the computer program product includes machine code that is directly executed by a processor or a computer, such as machine code produced by a compiler. In another example, the computer program product includes higher-level code that is executed by a processor or a computer using an interpreter. Programmers of ordinary skill in the art can readily write the instructions or software based on the block diagrams and the flow charts illustrated in the drawings and the corresponding descriptions in the specification, which disclose algorithms for performing the operations of the methods as described above.

The memory 201 may be a computer readable storage medium comprising instructions which, when the program is executed by a computer, cause the computer to carry out the steps of the methods according to any one of the above-described embodiments.

According to one embodiment, the computer-readable storage medium is a non-transitory computer-readable storage medium.

Computer programs implementing the methods of the present embodiments can commonly be distributed to users on a distribution computer-readable storage medium such as, but not limited to, an SD card, an external storage device, a microchip, a flash memory device, a portable hard drive and software websites. From the distribution medium, the computer programs can be copied to a hard disk or a similar intermediate storage medium. The computer programs can be run by loading the computer instructions either from their distribution medium or their intermediate storage medium into the execution memory of the computer, configuring the computer to act in accordance with the methods of this disclosure. All these operations are well- known to those skilled in the art of computer systems.

The instructions or software to control a processor or computer to implement the hardware components and perform the methods as described above, and any associated data, data files, and data structures, are recorded, stored, or fixed in or on one or more non-transitory computer-readable storage media. Examples of a non-transitory computer-readable storage medium include read-only memory (ROM), random-access memory (RAM), flash memory, CD- ROMs, CD- Rs, CD+ Rs, CD- RWs, CD+ RWs, DVD-ROMs, DVD- Rs, DVD+ Rs, DVD- RWs, DVD+ RWs, DVD- RAMs, BD- ROMs, BD-Rs, BD- R LTHs, BD- Res, magnetic tapes, floppy disks, magneto-optical data storage devices, optical data storage devices, hard disks, solid-state disks, and any device known to one of ordinary skill in the art that is capable of storing the instructions or software and any associated data, data files, and data structures in a non-transitory manner and providing the instructions or software and any associated data, data files, and data structures to a processor or computer so that the processor or computer can execute the instructions. In one example, the instructions or software and any associated data, data files, and data structures are distributed over network-coupled computer systems so that the instructions and software and any associated data, data files, and data structures are stored, accessed, and executed in a distributed fashion by the processor or computer.

### Production method for producing a biological object

After predicting at least one specific characteristic of a phenotype of a biological object thanks to the methods previously described, it is interesting to produce the biological object in order to test and validate its characteristics. Thus, the present disclosure further relates to a production method for producing a biological object identified by at least one part of its molecular sequence, said method comprises the following steps:
receiving said at least one part of molecular sequence identifying said biological object;
predicting a viability of said biological object according to a method for predicting at least one characteristic of a phenotype of said biological object identified by said at least one part of molecular sequence according to any one of the embodiments described hereabove, wherein the at least one characteristics of the phenotype comprises the viability; and
producing said biological object if the viability meets at least one predetermined criterion.

Advantageously, said method may further comprise testing said biological object produced to validate a list of technical effects related to said biological object, such as, for example, at least one therapeutic effect.

### Therapy treatment method

Thanks to the methods previously described, it could be possible to design biological objects for special therapy treatment and evaluate some characteristics of their phenotype before deciding if these biological objects are worth producing to be tested and eventually used if the tests are successful, which would save precious time and money.

An example of application but not restrictive is a gene therapy treatment method with a viral virus defined by at least one part of its molecular sequence, the method comprising:
producing said viral virus according to a production method for producing a biological object identified by at least one part of its molecular sequence according to any one of the embodiments described hereabove, wherein said biological object is said viral virus; and
applying said viral virus to a patient requiring said gene therapy treatment.

Advantageously, the viral vector may be newly designed adeno-associated virus.

An example of therapeutic application could be the regulation of the Interleukin-4 pathway in microglial cells infiltrated into the retinal tissue of patients suffering from dry forms of Age-Related Macular Degeneration (AMD).

## Claims

1. A computer-implemented method for preparing a reduced training dataset for training at least one machine learning model for predicting at least one characteristic of a phenotype of a biological object identified by at least one part of its molecular sequence, said method comprising:
- receiving a library of molecular sequences of reference (S 100), each molecular sequence of reference being labelled with respect to said at least one characteristic of the phenotype of said biological object to predict;
- computing, for each molecular sequence of reference, a first list of descriptors (S200) related to physicochemical and geometrical properties of at least one constructed 3D structure of said molecular sequence of reference;
- computing, for each molecular sequence of reference, a second list of descriptors (S300), said second list of descriptors comprising molecular descriptors computed directly from said molecular sequence of reference;
- creating a training dataset by applying said first and second lists of descriptors to said library of molecular sequences of references (S400);
- reducing the number of descriptors by selecting from said first and second lists of descriptors at least one reduced list of descriptors according to at least one first criterion and by using said training dataset (S500);
- generating said reduced training dataset by applying said at least one reduced list of descriptors to said library of molecular sequences of reference (S600); and
- outputting said reduced training database for training at least one machine learning model for predicting at least one characteristic of a phenotype of a biological object identified by at least one part of its molecular sequence (S650).

2. The method according to claim 1, wherein generating said first list of descriptors (S200) comprises for each molecular sequence of reference:
- constructing said at least one constructed 3D structure of said molecular sequence of reference;
- computing a first list of physicochemical and geometrical descriptors of said at least one constructed 3D structure,; and
- outputting said first list of descriptors which comprises said first lists of physicochemical and geometrical descriptors computed for each molecular sequence of reference.

3. The method according to claim 1 or 2, wherein computing said second list of descriptors (S300) comprises, for each molecular sequence of reference:
- aligning said molecular sequence of reference with a full-length sequence of reference;
- calculating molecular descriptors usingsaid aligned molecular sequence of reference constituting said second list of descriptors; and
- outputting said second list of descriptors which comprises all the calculated molecular descriptors.

4. The method according to any one of claims 1 to 3, wherein said at least one first criterion (S500) is one of or a combination of:
- a near-zero variance criterion, wherein near-zero variance descriptors, having a near zero variance, are excluded from said at least one reduced list of descriptors;
- a correlation criterion, wherein, whenever at least two descriptors are correlated within a predefined threshold, only one among said at least two descriptors is included insaid at least one reduced list of descriptors;
- a high variability criterion, wherein highly variable descriptors, representative of variability, are included in said at least one reduced list of descriptors, using an unsupervised machine learning receiving said training dataset as input.

5. The method according to any one of claims 1 to 4, wherein said biological object is an adeno-associated virus mutant and said library of molecular sequences is an adeno-associated virus capsid library.

6. The method according to claim 5, wherein said at least one characteristic of the phenotype predicted is a viability of said adeno-associated virus mutant.

7. A computer-implemented method for generating at least one trained machine learning model for predicting at least one characteristic of a phenotype of a biological object identified by at least one part of its molecular sequence, said method comprising:
- receiving a reduced training dataset (S700) prepared according to a method for preparing said reduced training dataset according to any one of claims 1 to 6;
- generating at least one machine learning model configured to receive said at least one part of molecular sequence identifying said biological object (S750);
- training the at least one generated machine learning model with said reduced training dataset (S900); and
- outputting said at least one trained machine learning model (S 1200), and optionally outputting automatically a list of statistics at the end of the training.

8. The method according to claim 7, wherein generating at least one machine learning model comprises generating at least two machine learning models of different types, each type of machine learning model being trained on said reduced training dataset and outputting a list of statistics representative of the performances of each trained machine learning model, the method further comprising:
- selecting, between the at least two trained machine learning model, at least one trained machine learning model having statistics satisfying a predetermined list of criteria computed at the end of the training step (S900); and
wherein the at least one trained machine learning model outputted at the end of said method (S 1200) is said at least one selected machine learning modelamong the at least two trained machine learning models.

9. The method according to claim 7 or 8, wherein, before training said at least one generated machine learning model (S900), further comprising:
- verifying that the scope of said reduced training dataset prepared is in a space of prediction of said at least one generated machine learning model (S800).

10. The method according to any one of claims 7 to 9, further comprising optimizing said reduced training dataset (S 1000) wherein an optimized list of descriptors is selected and applied to said library of molecular sequences of references to create an optimized reduced training dataset, said optimized list of descriptors being selected after a series of training sessions on untrained copies of said at least one generated machine learning model and comparisons of the list of statistics obtained after each training session.

11. The method according to any one of claims 7 to 10, further comprising a machine learning optimization (S 1100), wherein predetermined parameters of said at least one generated machine learning model are iteratively optimized by performing a series of training sessions on untrained copies of said at least one generated machine learning model and comparisons of the list of statistics obtained after each training session.

12. A computer-implemented method for predicting at least one characteristic of a phenotype of a biological object identified by at least one part of its molecular sequence, said method comprising:
- generating at least one trained machine learning model according to a method for generating said at least one trained machine learning model according to in any one of claims 7 to 11 (S10);
- receiving and preprocessing said at least one part of molecular sequence by applying the at least one reduced list of descriptors or the optimized list of descriptors when available (S20);
- inputting the at least one preprocessed part of molecular sequence into said at least one trained machine learning model or into the at least one selected and trained machine learning model when available (S30); and
- outputting said predicted at least one characteristic of the phenotype of said biological object (S40).

13. A computer program product comprising instructions which, when the program is executed by a computer, cause the computer to automatically carry out:
- a method for preparing a training dataset for training at least one machine learning model for predicting at least one characteristic of a phenotype of a biological object identified by at least one part of its molecular sequence according to any one of claims 1 to 6; and/or
- a method for generating at least one trained machine learning model for predicting at least one characteristic of a phenotype of a biological object identified by at least one part of its molecular sequence according to any one of claims 7 to 11; and/or
- a method for predicting at least one characteristic of a phenotype of a biological object identified by at least one part of its molecular sequence according to claim 12.

14. A non-transitory computer readable storage medium comprising instructions which, when the storage medium is read by a computer, cause the computer to automatically carry out:
- a method for preparing a training dataset for training at least one machine learning model for predicting at least one characteristic of a phenotype of a biological object identified by at least one part of its molecular sequence according to any one of claims 1 to 6; and/or
- a method for generating at least one trained machine learning model for predicting at least one characteristic of a phenotype of a biological object identified by at least one part of its molecular sequence according to any one of claims 7 to 11; or
- a method for predicting at least one characteristic of a phenotype of a biological object identified by at least one part of its molecular sequence according to claim 12.

15. A production method for producing a biological object identified by at least one part of its molecular sequence, said method comprising:
- receiving said at least one part of molecular sequence identifying said biological object;
- predicting a viability of said biological object according to a method for predicting at least one characteristic of a phenotype of said biological object identified by said at least one part of molecular sequence according to claim 12, wherein said at least one characteristics of the phenotype comprises the viability; and
- producing said biological object if said viability meets at least one predetermined criterion.
